# EUROPÄISCHE PATENTANMELDUNG

(11) **EP 1 243 649 A1**
(43) Veröffentlichungstag der Anmeldung: **25.09.2002**
(21) Anmeldenummer: 01107318.6
(22) Anmeldetag: 23.03.2001
(51) Int. Cl.: C12N 15/10

(54) **Verfahren zur Isolierung von DNA**

(71) Anmelder: GeneScan Europe AG, 79108 Freiburg (DE)
(72) Erfinder: Müller, Werner, Prof. Dr., 64646 Heppenheim (DE)
(74) Vertreter: Teipel, Susanne, Dr.

(57) **Zusammenfassung**

Die Erfindung betrifft ein Verfahren zur Isolierung und Größenfraktionierung von DNA aus einem Probenmaterial, bei dem man das DNA-haltige Probenmaterial mit einem alkalischen Medium behandelt, an einen speziellen, stark basischen Anionenaustauscher selektiv bindet und die DNA, gegebenenfalls nach der Größe fraktioniert, eluiert.

## Beschreibung

Die Erfindung betrifft ein Verfahren zur Isolierung von einzelsträngiger DNA aus einem Probenmaterial, bei dem man das DNA-haltige Probenmaterial mit einem stark alkalischen Medium behandelt bzw. aufschließt, die DNA in diesem Medium an einen speziellen, stark basischen Anionenaustauscher bindet und diese nach Entfernen von anderen gebundenen Probenbestandteilen, gegebenenfalls nach der Größe fraktioniert, eluiert.

Für die Isolierung von DNA, beispielsweise aus Nahrungsmitteln zur Herkunftsbestimmung der Komponenten, stehen etliche Verfahren zur Verfügung. Welches zur Anwendung kommt, hängt meist von der Art der Probe ab. Ziel ist stets, genügend DNA in ausreichender Qualität zu isolieren, um eine Amplifikation spezifischer Gen-Abschnitte mittels der PCR-Technik zu ermöglichen. Trotz aller zur Verfügung stehenden Verfahren ist dieses Ziel bei etlichen Proben nicht oder nur schwer zu erreichen.

Uberraschenderweise wurde festgestellt, daß spezielle, stark basische Anionenaustauscher aus einem stark alkalischen Medium einzelsträngige DNA selbst bei erhöhter Ionenstärke (z.B. 0.5 M Kochsalz) binden können und bei der Elution mit höheren Salzkonzentrationen die Größenselektivität der DNA-Bindung beibehalten, die man von Anionenaustauschern beim Arbeiten in neutralen Medien für doppelsträngige DNA kennt.

Die Erfindung betrifft somit ein Verfahren zur Isolierung (und gegebenenfalls Größenfraktionierung) von einzelsträngiger DNA aus einem Probenmaterial, bei dem man
a) das DNA-haltige Probenmaterial mit einem alkalischen Medium bei pH > 12, gegebenenfalls nach Vorbehandlung mit einem oder in Gegenwart eines geeigneten grenzflächenaktiven und/oder chaotropen Mittel, behandelt,
b) in diesem Medium mit einem speziellen, stark basischen Anionenaustauscher in Kontakt bringt, welcher als bindende Gruppen quartäre Alkylammonium-Reste (die im folgenden auch als Tetraalkylammonium- bzw. Trialkylammoniumalkyl-Reste bezeichnet werden) enthält, die in unvernetzten hydrophilen Linear-Polymeren eingebaut sind, die ihrerseits auf der Oberfläche inerter Trägerteilchen kovalent fixiert sind, und
c) die an den Anionenaustauscher gebundene DNA im alkalischen Medium von diesem eluiert.

Die wesentliche Vorausetzung für das Verfahren ist die überraschende Tatsache, daß die Phosphatgruppen in der DNA eine mindestens 9 Größenordnungen höhere Affinität zu den quartären Tetraalkylammoniumgruppen des Austauschers zeigen, als Chlorid- und Hydroxylionen. Möglicherweise rührt dieser Effekt z.T. daher, daß die basischen Gruppen des zur Anwendung kommenden speziellen Austauschers in einer linearen Anordnung auf aufgepfropften Linear-Polymeren vorliegen, die sich zu den Phosphatgruppen der DNA in passenden Abständen anordnen können. Damit wäre die hohe Affinität der basische Gruppen des Austauschers für die DNA-Phosphatreste wenigstens z.T. kooperativen Ursprungs.

Durch die Anwendung des erfindungsgemäßen Verfahrens läßt sich bei der Probenaufbereitung bzw. -vorbereitung die normalerweise vor der eigentlichen DNA-Isolierung erforderliche Abtrennung von Störkomponenten wie Proteine, Fette und RNA weitgehend vermeiden. Insbesondere entfällt die Abscheidung denaturierter Proteine und ähnlicher Stoffe durch Ausschütteln mit agressiven oder gesundheitschädlichen organischen Lösungsmitteln wie Phenol oder Chlorofom, die meist erhebliche Verluste an DNA verursachen.

Die fast immer im Überschuß zur DNA vorliegende RNA wird unter den alkalischen Bedingungen völlig abgebaut, was den sonst üblichen RNAse-Abbau überflüssig macht.

Insbesondere wird die DNA, die je nach thermischer Vorgeschichte des Untersuchungsmaterials meist in denaturierter Form vorliegt, durch die Alkalibehandlung bzw. den Alkaliaufschluß in eine gut lösliche Form überführt. Dies ist insofern wichtig, als einzelsträngige DNA in neutralen Medien schon bei Ionenstärken um 0.1 unlöslich ist und eine hohe Tendenz zur unspezifischen Adsorption an Oberflächen aller Art zeigt.

Ein weiterer Vorteil des Verfahrens ist die im alkalischen Medium erfolgende Entladung der basischen Gruppen der Histone, was eine erhebliche Erleichterung der DNA-Extraktion darstellt, da die freigesetzten Histon-Proteine bei der nachfolgenden Bindung der DNA an den Anionenaustauscher bei pH > 12, wenn überhaupt, dann nur sehr schwach gebunden werden und sich somit leicht entfernen lassen.

Weitere vorteilhafte und/oder bevorzugte Ausführungsformen der Erfindung sind Gegenstand der Unteransprüche.

Nach einer Ausführungsform der Erfindung enthält das alkalische Medium für die Behandlung bzw. den Aufschluß des DNA-haltigen Probenmaterials Alkalihydroxid wie z.B. NaOH oder KOH in Konzentrationen im Bereich von 0.1 M bis 1 M.

Nach einer weiteren Ausführungsform der Erfindung wird zur Elution der DNA von dem erfindungsgemäß verwendeten Anionenaustauscher als Trennmaterial ein Salz wie beispielsweise NaCl oder KCl in einer Konzentration im Bereich von 0.6 bis 1,2 M, vorzugsweise im Bereich 0.7 bis 0.9 M, oder ein Salzgradient im Bereich von 0.6 bis 1.2 M verwendet.

Das erfindungsgemäße Verfahren ist besonders zur Isolierung und gegebenenfalls zur Größenfraktionierung von DNA aus Nähroder Nahrungsmitteln pflanzlichen oder tierischen Ursprungs wie Kartoffeln, Tomatenmark, Orangensaft-Konzentrat, Lecithin, Schokolade, Nougat-Creme, Mais- oder Sojamehl, Fleischund Wurstprodukten aller Art geeignet. Darüber hinaus ist das Verfahren besonders zur raschen Isolierung von DNA aus Bakterien und anderen Mikroorganismen wie E. coli geeignet.

Im folgenden wird die Erfindung ohne Beschränkung anhand von Ausführungsbeispielen detaillierter beschrieben.

Das für die Behandlung bzw. den Aufschluß des DNA-haltigen Probenmaterials verwendete alkalische Medium unterliegt hinsichtlich des Basentyps keiner besonderen Einschränkung. Die Basizität muß lediglich hoch genug sein, um einen pH-Wert über 12 zu gewährleisten. Somit könnten auch Erdalkalihydroxide Anwendung finden, soweit sie nicht schwer- oder unlösliche Salze mit Nucleinsäuren oder anderen Probenkomponenten bilden, die zu Störungen des Analysenablaufes oder sogar zum Verlust der DNA führen könnten. Von den organischen Basen kämen hinsichtlich der Basizität nur quartäre Ammonium-Basen anstelle von Alkalihydroxiden in Betracht; die hohe Affinität der Tetraalkylammonium-Kationen zu den Phosphatresten, die man bei der Bindung der DNA an den Anionenaustauscher ausnutzt, würde aber zu den Austauschergruppen in Konkurrenz treten und damit die Bindung an den Austauscher beeinträchtigen. NaOH oder KOH werden einmal aus diesem Grunde, zum anderen wegen ihrer Verfügbarkeit in jedem Labor und ihres geringen Preises bevorzugt. Der Konzentrationsbereich, in dem das Alkali zur Anwendung kommt, wird nach unten hin durch den Mindest-pH-Wert bestimmt, der erforderlich ist, um alle organischen Basen (außer den quartären Ammonium-Basen) zu entladen bzw. das Probenmaterial gut aufzuschließen. Nach oben hin ist die Alkali-Konzentration insofern begrenzt, als eine Desaminierung der Nucleobasen in der DNA vermieden werden sollte. Der oben angegebene Konzentrationsbereich von 0.1 bis 1.0 M hat sich bewährt, könnte aber auch je nach Probematerial unter- oder überschritten werden.

Es ist klar, daß festes und/oder grobes Probenmaterial vor der alkalischen Behandlung bzw. vor dem alkalischen Aufschluß auf geeignete Weise zerkleinert werden kann, beispielsweise durch Zerdrücken, Zerhacken oder Zermahlen, und daß gegebenenfalls in dem alkalischen Behandlungsmedium unlösliche Rückstände entfernt werden können, beispielsweise durch Zentrifugation oder Filtration. Ferner kann es zweckmäßig sein, beispielsweise bei stark fetthaltigen Proben, Entfettungsschritte (z.B. durch Behandlung mit Lösungsmitteln) oder bei überwiegend aus z.B. Stärke bestehenden Proben, enzymatische Abbaureaktionen, z.B. mit Amylase, durchzuführen, um die weitere Probenhandhabung zu erleichtern. Störende Begleitproteine können z.B. durch Behandlung mit Proteasen entfernt werden. Verfahren zur Entfettung bzw. zum enzymatischen Abbau von Polysacchariden wie Stärke oder Proteinen sind im Stand der Technik in großer Zahl bekannt und werden vom Fachmann nach Maßgabe der praktischen Gegebenheiten ausgewählt und angewendet. Diese Vorbehandlungen müssen nicht bei pH > 12 durchgeführt werden.

Ferner kann eine Vorbehandlung mit einem oder mehreren geeigneten grenzflächenaktiven und/oder chaotropen Mittel erfolgen, wenn das alkalische Medium alleine nicht zum oder zum vollständigen Aufschluß der Probe, d.h. zur quantitativen Freisetzung und Erfassung der gesamten vorhandenen DNA, ausreicht. Darüber hinaus kann auch in dem alkalischen Medium zur Behandlung ein (oder mehrere) geeignetes grenzflächenaktives und/oder chaotropes Mittel enthalten sein. Falls mehrere grenzflächenaktive und/oder chaotrope Mittel verwendet werden, können diese gleich oder verschieden sein. Diese Vorbehandlungen müssen nicht bei pH > 12 durchgeführt werden.

Die erfindungsgemäße Behandlung mit dem alkalischem Medium bei pH > 12 kann somit gleichzeitig mit einem (alkalischen) Aufschluß der Probe verbunden sein oder umfaßt, nach Maßgabe der praktischen Gegebenheiten, auch entsprechende Probenvorbehandlungen zum oder zur Unterstützung des Aufschlußes, beispielsweise die oben erwähnten.

Als Trennmaterial wird in dem erfindungsgemäßen Verfahren ein spezieller, stark basischer Anionenaustauscher verwendet.

Dieser Anionenaustauscher besteht aus porösen oder unporösen, durch Hydroxylfunktionen inertisierte Partikeln, auf deren Oberfläche hydrophile Linear-Polymere aufgepfropft sind. In diese Polymere sind quartäre Alkylammoniumgruppen (die auch als Tetraalkylammoniumgruppen bzw. Trialkylammoniumalkylgruppen bezeichnet werden) wie Trimethylammoniumalkyl(z.B. ethyl)gruppen über den Alkylrest in regelmäßigen oder unregelmäßigen Abständen eingebaut. Trennmaterialien dieses Typs sowie ihre Herstellung werden detailliert in der europäischen Patentschrift 0 337 144 B1 (Merck Patentgesellschaft mbH) beschrieben, auf die hier ausdrücklich Bezug genommen wird, bzw. sind auch unter dem Handelsnamen Fractogel EMD TMAE (TMAE steht für Tetramethylammoniumethyl) (Normal- oder Hicap-Typ) bei der Firma Merck KGaA, Darmstadt, Deutschland erhältlich.

Als funktionelle Gruppen der aufgepfropften Polymerketten, den sog. »Tentakeln«, kommen nur quartäre Ammonium-gruppierungen in Betracht, da andere basische Gruppen bei dem pH der Anwendung entladen werden und damit keine elektrostatische Wechselwirkung mit der DNA mehr eingehen können. Die Liganden am Stickstoffatom der quartären Ammoniumgruppen sollen aus C₁- oder C₂-Einheiten bestehen, um überlagernde hydrophobe Effekte zu vermeiden. Diese würden die Spezifität der DNA-Bindung drastisch senken. Aufgrund der Beweglichkeit der »Tentakel« können die polyanionischen DNA-Moleküle entsprechende polykationische Bindungsstellen im optimalen Abstand zur festen Matrix der Trägerteilchen finden, wodurch eine unspezifische Adsorption der DNA an der Oberfläche der Trägerteilchen vermieden wird.

Vorzugsweise befindet sich das Trennmaterial in einer Chromatographie-Säule, durch welche die DNA-haltige alkalische Lösung des Probenaufschlusses meist bei geringem Überdruck durchgeleitet wird. Durch diese Arbeitsweise wird die vollstandige Erfassung der DNA am besten gewährleistet.

Zur Bindung der DNA kann das Trennmaterial aber auch in die alkalische Probelösung eingebracht werden und die Bindung durch Rühren oder durch Schütteln bewerkstelligt werden. Solch ein Adsorptionsprozeß dauert allenfalls etwas länger als die DNA-Bindung in der Säule.

In beiden Fällen könnte die Bindung der DNA an den Austauscher photometrisch verfolgt werden; meist scheitert dies jedoch einmal an den geringen Mengen der vorliegenden DNA, zum anderen an der UV-Absorption der Nebenbestandteile, die meist in erheblichem Überschuß in der Alkalibehandlungslösung vorliegen.

Es ist zweckmäßig, Fette und Polysaccharide vor der Alkalibehandlung aus der Probe zu entfernen, da sie wasserunlösliche Schichten bzw. Gele bilden, die das Trennmaterial in der Säule leicht verstopfen. Eine n-Hexan-Extraktion bzw. eine kurze Inkubation mit Amylasen oder andern geeigneten abbauenden Enzymen empfiehlt sich in solchen Fällen. Überschüssige Proteine stören weniger, da sie meist in Alkali gut löslich sind und bei weitem schwächer als die DNA vom Trennmaterial gebunden werden. Bei sehr hohen Mengen Begleitprotein bewährt sich eine kurze Vorinkubation der Probe mit Proteinase K in Anwesenheit von Natrium-Lauroylsarkosinat (SLS). Hinsichtlich niedermolekularer Stoffe stören nur große Mengen Di-oder Tricarbonsäuren, Phosphate und Guanidin-isothiocyanat. Alle lassen sich am einfachsten durch eine vorgeschaltete Gelfiltration aus der Alkalibehandlungslösung entfernen. Als Medien für diesen Schritt haben sich Sephadex® G25 (Pharmacia, Uppsala, Schweden) insbesondere aber das Fractogel EMD Bio-SEC (Merck, Darmstadt, Deutschland) bewährt.

Zur Elution der an das erfindungsgemäß verwendete Trennmaterial gebundenen DNA kann eine Lösung eines Salzes wie beispielsweise NaCl oder KCl verwendet werden. Hinsichtlich des verwendeten Salzes gibt es keine besonderen Beschränkungen, solange sich die zur Elution erforderliche Ionenstärke und ein pH-Wert > 12 einstellen lassen. Geeignete Salztypen und Konzentrationsbereiche lassen sich durch einfache Routineversuche ermitteln. NaCl oder KCl werden jedoch bevorzugt, weil sie billig sind, in praktisch jedem Labor zur Verfügung stehen und sich allgemein zur Elution von Austauschermaterialien bewährt haben.

Außerdem gestattet die Elution mit einem Salzgradienten auf einfache Weise eine Fraktionierung der gebundenen DNA nach der Größe. Beispielsweise lassen sich synthetische Oligonucleotide oder Restriktionsfragmente bis 50 Nucleotide Größe bei einer Auflösung von ± 2 Nucleotiden trennen, bis ca. 150 Nucleotide lassen sich Trennungen mit allerdings kontinuierlicher Abnahme der Auflösung durchführen. Bei längeren DNA-Stücken wird die Größentrennung von einer Selektionierung nach der Basenzusammensetzung überlagert, wobei G- und T-reiche Stränge später als A- und C-reiche eluiert werden. Diese Selektivität konnte erfolgreich zur Separation der komplementären Stränge eines Restriktionsfragmentes von 125 Basenpaaren im präparativen Maßstab bei < 0.5% Überlappung herangezogen werden.

Ein stufenweise ansteigendes Konzentrationsprofil wird erzeugt, indem nacheinander, d.h. chargenweise, Salzlösungen ansteigender Konzentration durch die Säule geführt werden, und ein kontinuierliches Konzentrationsprofil, vorzugsweise mit linearem Verlauf, unter Verwendung eines im Stand der Technik bekannten bzw. auch im Handel erhältlichen Gradientenmischers. Je nach Trennproblem kommen auch Mischprofile in Frage, d.h. mit teilweise stufenförmigem und teilweise kontinuierlichem Konzentrationsverlauf. Es ist klar, daß im Falle eines kontinuierlichen Konzentrationsverlaufes dieser nicht linear sein muß, sondern je nach Trennproblem beispielsweise auch exponentiell ansteigend oder im oberen Konzentrationsbereich abflachend verlaufen kann.

Wenn in dem erfindungsgemäßen Verfahren ein Stufengradient angewendet wird, geht man im allgemeinen von einer NaCl-Konzentration von 0.3 M im alkalischen Grundmedium (meist 0.3 M NaOH) aus, steigert dann die Konzentration auf 0.6 M NaCl und danach in Schritten von je 0.1 M NaCl bis 1.2 M. Die Elutionsschritte unterhalb 0.7 M und oberhalb 0.9 M dienen im allgemeinen nur der Elution von Fremdprodukten und kleinen Oligonucleotiden (< 20 Nucleotide), bzw. der Säulenreinigung oder Prüfung auf vollständige Elution der DNA.

Die Säule wird mit 0.3 M NaCl in 0.3 M NaOH konditioniert. Dies hat u.a. den Vorteil, daß ein großer Teil der sauren Probenbegleitstoffe ebenso wie die neutralen und basischen garnicht erst an den Anionenaustauscher gebunden werden.

Wie bereits oben ausgeführt ist das erfindungsgemäße Verfahren zwar besonders zur Isolierung und Größenfraktionierung von DNA aus Nähr- oder Nahrungsmitteln pflanzlichen oder tierischen Ursprungs geeignet, jedoch nicht darauf beschränkt. Grundsätzlich kann es auf beliebiges biologisches Material angewendet werden.

### Beispiele

Die folgenden Puffer wurden verwendet:
TE-Puffer: 10 mM Tris.HCl, 1 mM EDTA, eingestellt mit NaOH auf pH 8.0
Gua-Puffer: 10 ml 1 M Tris.HCl, pH 8.0; 8.8 ml 0.65 M ED-TA, pH 8.0; 2.6 g Triton X100; 120 g Guanidin-Isothiocyanat; 103.2 ml Wasser

### I. Ermittlung geeigneter Aufschlußmethoden und -medien

Im Zuge der Untersuchungen zeigte sich, daß bei weitem nicht alle tierischen oder pflanzlichen Produkte durch Alkali allein soweit aufgeschlossen werden können, daß vorhandene DNA weitgehend freigesetzt und erfaßt wird. In diesem Fall kann das alkalische Medium ein grenzflächenaktives und/oder chaotropes Mittel zur Solubilisierung enthalten. Auch kann eine kurze Vorbehandlung der Probe mit diesen Mitteln und/oder geeigneten Enzymen zum Abbau von Begleitpolysacchariden oder -proteinen wie Amylasen oder Proteinase K und/oder eine Lösungsmittelextraktion zur Entfernung von Begleitlipiden bei tieferen pH-Werten, d.h. vor der eigentlichen Alkalibehandlung, vorgenommen werden.

Beispielsweise erwies sich Lauroylsarcosinat (SLS) als ein geeignetes grenzflächenaktives Mittel bzw. Netzmittel, welches unter den üblichen Arbeitsbedingungen nicht vom verwendeten Anionenaustauscher gebunden wird.

Zum anderen bewährte sich als chaotropes Mittel beispielsweise Guanidin-isothiocyanat (»Gua«) schon in 2.5 M Konzentration als ein hochwirksames Solubilisierungsmittel für die meisten Proben biologischen Ursprungs. Leider zeigt das Isothiocyanat-Anion eine ungewöhnlich hohe Affinität zu den quartären Tetraalkylammoniumgruppen des Austauschers, was eine Abtrennung des Agens' durch einen Gelfiltrationsschritt vor der DNA-Bindung an den Austauscher erforderlich macht. Da dieser Schritt ohnehin in den meisten Fällen zur Entfernung niedermolekularer, potentiell störender Begleitstoffe durchgeführt wird, stellt somit die Affinität des Agens zum Austauscher keinen zusätzliches Problem dar. Ähnlich wirksam wie Guanidin-isothiocyanat erwies sich nur Formamid. Es schien besonders attraktiv, weil es keinen Einfluß auf die Ionenstärke des Aufschlußmediums ausübt und beim Austauscherschritt vollständig entfernt wird. Leider stellte sich jedoch heraus, daß die Polymerase-Kettenreaktion entweder gegenüber dem Agens selbst oder einer begleitenden Verunreinigung so empfindlich ist, daß sich die Verwendung dieses Solubilisierungsmittels ausschloß.

Mechanische Hilfsmittel zum Dispergieren der Proben sind selten erforderlich. Beim Aufschluß von Fleisch- oder Wurstproben bewährte sich ein Ultraturrax®-Homogenisator der Fa. Haake, Berlin. Festes Naßgut wie z.B. Kartoffeln werden entweder im Homogenisator mit rotierenden Messern zerkleinert oder mit Seesand im Porzellanmörser unter Kohlendioxid oder Stickstoff zerrieben.

### II. Ermittlung der Mindestaffinität des Fracto-EMD-TMAE-Austauschers für DNA

DNA aus Putenleber wurde in einer Konzentration von 500 ng/ml in 0.3 M NaCl/0.3 M NaOH zunächst durch ein Bett aus ca. 5 x 1.3 cm Sephadex®-Gel (Pharmacia) oder BioSEC-Gel (Merck) im gleichen Medium filtriert, um niedermolekulare Probenbestandteile zu entfernen, und sofort durch ein Bett aus ca. 5 x 5 mm TMAE-Trennmaterial (100 µl TMAE-Trennmaterial in einem Microspin(Pharmacia)-Säulchen geleitet. Die TMAE-Schicht wurde mit 0.5 ml des gleichen Mediums gespült und die gebundene bzw. sorbierte DNA mit 80 µl 1.5 M NaCl/0.3 M NaOH eluiert. Das durch Zentrifugation gewonnene Eluat wurde durch Filtration über Sephacryl® S300 (Pharmacia) im Microspin-System in TE-Puffer überführt und im Spektralphotometer (Mikroküvette, 200 µl Lösung) vermessen. Da 10 µg DNA in 20 ml aufgegeben wurden, sollten bei vollständiger Bindung der DNA unter Berücksichtigung der Denaturierung (Hyperchromie von 20%) ca. 1.2 OD_{~260} gemessen werden. Gefunden wurden 1.183 OD₂₅₈. Dieses Ergebnis bedeutet, daß die aufgegebene DNA-Konzentration noch deutlich oberhalb der Gleichgewichtskonzentration für das DNA-TMAE-Bindungsgleichgewicht lag.

In einem weiteren Versuch wurde daher die DNA-Konzentration in der Aufgabelösung auf 50 ng/ml gesenkt und der Test in der beschriebenen Weise wiederholt. Mit dem Photometer sollten 0.120 OD_{~260} gemessen werden. Das Spektrum zeigte am Maximum eine Absorption von 0.117, was bedeutet, daß selbst diese Konzentration von fast 10⁻⁷ Mol/l (bezogen auf Mononucleotid oder DNA-Phosphat) noch quantitativ sorbiert wurde. Die Dissoziationskonstante der Bindung muß somit deutlich unter 10⁻⁸ mol/l liegen. Anschaulicher ausgedrückt bedeutet dies, daß 5 ng/ml DNA (10⁻⁸ M) noch mindestens zu 90% unter den angegeben Bedingungen an das TMAE-Trennmaterial gebunden werden.

### III. DNA-Isolierung aus Leber und Wurst

### 1. Aufschluß des Probenmaterials

0.5 g Probenmaterial wurden in Gua-Puffer (ohne Triton X100) durch Schütteln dispergiert und mit NaOH auf 0.3 M eingestellt. Zur Homogenisierung kann gegebenenfalls ein Ultraturrax®-Handgerät eingesetzt werden. Das Homogenisat wurde 5 min im Wasserbad auf 95-100°C erhitzt und danach zentrifugiert (Hettich-Zentrifuge, Ausschwingrotor 1624, 4000 UpM, 10 min).

### 2. Abtrennung von niedermolekularen Komponenten und Bestimmung der DNA-Bindung:

Der Überstand (ca. 2 ml) wurde ohne weitere Behandlung über eine geeignete Gelfiltrationssäule (z.B. 4.5 x 1.3 cm, bevorzugte Füllung: BioSEC-Gel, Merck) in 0.3 M NaCl/0.3 M NaOH filtriert, an die eine weitere Säule mit TMAE-Trennmaterial (Merck) (100 µl in einer Microspin-Säule) angeschlossen war. An dieser TMAE-Säule werden die hochmolekularen Probenbestandteile »Online« gebunden bzw. sorbiert.

### 3. Elution bzw. Desorption der DNA, Entsalzung, spektralanalytische Untersuchung und Amplifikation durch PCR

Nach Waschen des der TMAE-Säule mit 0.3 M NaCl/0.3 M NaOH wurde die gebundene DNA mit einem »Salzstoß«, d.h. mit 80 µl 1.5 M NaCl/0.3 M NaOH desorbiert. Das Eluat ließ sich durch Mikro-Gelfiltration im Microspin-System in TE-Puffer überführen. Von den erhaltenen Lösungen werden UV-Spektren in der Mikroküvette aufgenommen und nach weiteren Verdünnungen in PCR-Experimenten eingesetzt.

### 4. Ergebnisse

Aus 0.5 g Rinderleber wurden 8.12 OD₂₅₈ isoliert, was einer DNA-Menge von 0.6 mg entspricht. Auf dem Agarosegel zeigte sich eine hohe Polydispersität dieser DNA mit überwiegend kleineren Fragmenten (< 10⁶ D).

Aus 0.4 g Brühwurst wurden 1.32 OD, entsprechend 61 µg DNA, erhalten, in der größere Fragmente deutlich überwogen.

Nach Restriktionsabbau lieferte die Polymerase-Kettenreaktion mit artspezifischen Primern große Mengen Amplifikat.

### IV. Isolierung von DNA aus Tomatenmark, Orangensaft-Konzentrat, Lecithin, Schokolade, Nougat-Creme und Maisstärke.

Die Isolierung amplifizierbarer DNA aus diesen Materialien gilt als problematisch, was entweder auf die geringen DNA-Mengen in den Proben oder auf störende Inhaltstoffe dieser Materialien zurückzuführen ist. Letzteres trifft insbesondere für Tomatenmark, Orangensaftkonzentrate und Schokoladenprodukte zu. Die Beseitigung dieser Probleme ist durch die Anwendung des erfindungsgemäßen Verfahrens unter Elution der an das TMAE-Trennmaterial gebundenen hochmolekularen sauren Nebenbestandteile durch Eluieren der DNA in einem engen »Salzfenster« möglich.

### 1. DNA aus Tomatenmark

0.5 g Tomatenmark wurden in 1.5 ml Gua-Puffer (ohne Triton X100) suspendiert, mit 80 µl 8 M NaOH auf ca. 0.3 M NaOH eingestellt, 5 min im Wasserbad auf 95-100°C erhitzt und nach dem Abkühlen 10 min bei 4000 UpM (Hettich, Rotor 1624) zentrifugiert. 2 ml des Überstandes wurden durch die oben in III. 2. beschriebene BioSEC-Säule filtriert. Nur die ersten 3 ml des Durchlaufs wurden durch die TMAE-Säule geleitet. Nach 2.5 ml verfärbte sich der Durchlauf schwach rötlich, was zu einer Orangefärbung des TMAE-Bettes führte.

Im ersten Ansatz wurden die an das TMAE-Trennmaterial gebundenen sauren Polymere »en bloc« mit 0.3 M NaCl/1.5 M NaOH desorbiert. Die Produkte ergaben nach Microspin-Umpufferung ein Spektrum, welches kaum einen Hinweis auf isolierte DNA lieferte. Eine PCR-Amplifikation war ebenfalls nicht möglich.

In einem weiteren Isolierungsversuch mit 1 g Tomatenmark wurden die an das TMAE-Trennmaterial gebundenen Materialien durch stufenweise Erhöhung der Salzkonzentration zum Teil fraktioniert. Dabei zeigte sich, daß die Komponente, welche im UV-Spektrum dominierte, bei einer Konzentration von 0.5 M NaCl/0.3 M NaOH eluiert. Komponenten mit für DNA typischen UV-Spektren wurden bei einer Konzentration von 0.8 M und 0.9 M NaCl, jeweils in 0.3 M NaOH, eluiert. Bei Erhöhung der Salzkonzentration auf 1.0 bzw. 1.2 M NaCl, jeweils in 0.3 M NaOH, wurden dann wieder Komponenten eluiert, deren Spektren mehr den Spektren der mit 0.5 M NaCl erhaltenen Eluate ähnelten.

Die mit 0.8 M und 0.9 M NaCl (jeweils in 0.3 M NaOH) erhaltenen Eluate lieferten problemlos PCR-Amplifikate, die mit 0.5 M NaCl erhaltenen Eluate dagegen nicht, sie hemmten sogar die Amplifikation zugesetzter DNA.

### 2. DNA aus Orangensaft-Konzentrat

2 g Orangensaft-Konzentrat wurden mit 140 µl 8 M NaOH neutralisiert, mit 2.0 ml Gua-Puffer (ohne Triton X100) vermischt, mit 133 µl 8 M NaOH auf ca. 0.3 M NaOH eingestellt, 5 Min. auf 90-100°C erhitzt und 10 Min bei 4000 UpM (Hettich, Rotor 1624) zentrifugiert, worauf 3 ml des Überstandes (ca. 90%) auf die oben in III. 2. beschriebene BioSEC/TMAE-Säulenkombination gegeben und mit leichtem Überdruck durchgedrückt wurden. Anschließend wurde mit 1 ml 0.3 M NaCl/0.3 M NaOH nachgespült. Das gelb gefärbte TMAE-Bett wurde zuerst 2 x mit je 0.4 ml 0.3 M NaCl/0.3 M NaOH gewaschen und dann 2 x mit je 80 µl 0.7 M NaCl/0.3 M NaOH eluiert. Die gelben Eluate lieferten nach dem Umpuffern in TE-Puffer mit dem Microspin-System Spektren gelber Farbstoffe mit charakteristischen Banden zwischen 380 und 500 nm, im UV eine deutliche Bande um 278 nm, aber keine Bande um 260 nm, die auf DNA hingewiesen hätte. Die weiteren 2 Elutionen mit je 80 µl 0.9 M NaCl/0.3 M NaCl lieferten Eluate mit eindeutigen DNA-Spektren. Die erste Elution zeigte im Spektrum ca. 0.08 OD entspr. 4 µg/ml DNA oder 0.8 µg in 0.2 ml. Die zweite Elution ergab weitere 0.4 µg in 0.2 ml.

Beide DNA-Fraktionen wurden über Microspin-Säulchen in TE-Puffer umgepuffert.

Die Polymerase-Kettenreaktion lieferte reichlich Amplifikate.

### 3. DNA aus Lecithin

1 g Lecithin wurden in 1.5 ml Gua-Puffer (ohne Triton X100) und 10 ml Hexan geschüttelt, worauf 1 ml Gua-Puffer zusätzlich zugegeben und danach 15 min in der Hettich-Zentrifuge (Rotor 1624) bei 4000 UpM zentrifugiert wurde. Die Hexanschicht wurde abgegossen, die Lipidschicht durchstochen und die wäßrige Phase herauspipettiert (1.2 ml). Nach Zusatz von 80 µl 8 M NaOH wurde die Lösung mit geringem Unterdruck durch die oben in III. 2. beschriebene BiOSEC/TMAE-Säulenkombination gesaugt (der lineare Fluß wurde auf ca. 1 cm/min eingestellt), worauf mit 1.5 ml 0.3 M NaCl/0.3 M NaOH nachgespült wurde. Insgesamt passierte ein Volumen von 2.5 ml die TMAE-Säule. Diese wurde dann mit 0.4 ml 0.3 M NaCl/0.3 M NaOH gespült und mit jeweils 80 µl 0.7 M NaCl, 0.9 M NaCl und 1.2 M, jeweils in 0.3 M NaOH, eluiert. Nach dem Umpuffern in TE-Puffer in Microspin-Säulchen wurden die UV-Spektren gemessen.

Die Spektren zeigten, daß sich die Hauptmenge der DNA (ca. 5 µg) in der ersten, d.h. mit 0.7 M NaCl erhaltenen, Elutionsfraktion befand. Dies ist nur verständlich, wenn die DNA im Lecithin aus relativ kleinen Fragmenten besteht. Mit 0.9 M NaCl wurden ca. 1 µg DNA erhalten und mit 1.2 M NaCl weitere 0.5 µg. Nach den Spektren, befand sich in der 2. Fraktion (0.9 M NaCl) die reinste DNA.

Alle drei Fraktionen ergaben erwartungsgemäß PCR-Amplifikationsprodukte.

### 4. DNA aus Schoko-Nougat-Creme

1 g Nuß-Nougat wurden in 1.5 ml Gua-Puffer (mit Triton X100) durch Schütteln dispergiert und 10 min bei 4000 UpM zentrifugiert (Hettich, Rotor 1624), wobei sich eine farblose Ölphase, eine rotbraune wäßrige Phase und ein schwarzes Sediment bildeten. Die Ölphase und die wäßrige Phase wurden getrennt abgehoben. Das Sediment wurde erneut mit 1 ml Gua-Puffer dispergiert und wie zuvor abzentrifugiert. Die beiden Gua-Extrakte wurden vereinigt (Gesamtmenge 2.2 ml) und mit 100 µl 8 M NaOH versetzt, wobei sich die Lösung nach dunkler verfärbte. Nach erneuter Zentrifugation (wie oben beschrieben) wurde der dunkelbraune Überstand abgehoben und in 0.3 M NaCl/0.3 M NaOH unter leichtem Überdruck über die oben in III. 2. beschriebene BiOSEC/TMAE-Säulenkombination filtriert. Nach dem Nachspülen mit 1 ml des gleichen Mediums (wobei geringe Mengen braungefärbter Anteile die BioSEC-Säule passierten) wurde die TMAE-Säule abgehängt, mit 0.5 ml 0.3 M NaCl/0.3 M NaOH gewaschen und mit jeweils 80 µl 0.7 M NaCl, 0.9 M NaCl und 1.2 M NaCl, jeweils in 0.3 M NaOH, eluiert. Die Eluate wurden über Microspin-Säulchen in TE-Puffer überführt und mit dem Spektralphotometer vermessen. Alle drei Fraktionen enthielten Komponenten, die bei etwa 260 nm absorbierten. Diese Absorption war bei der mit 0.9 M NaCl eluierten Fraktion am ausgeprägtesten. Bei der dritten Fraktion (1.2 M NaCl) überwogen die bei 280 nm absorbierenden Komponenten.

Alle drei Fraktionen lieferten deutlich erkennbar PCR-Amplifikate.

Der nach der zweiten Extraktion verbliebene Rückstand wurde mit 1.5 ml Gua-Puffer (ohne Triton X100) und 100 µl 8 M NaOH resuspendiert und 5 min auf 95-100°C erhitzt. Nach Zentrifugation (wie oben) wurde die fast schwarze Lösung wie oben beschrieben aufgearbeitet. Die Spektren zeigten mit deutlichen Schultern um 280 nm überwiegend homocyclische Aromaten an.

Die Ergebnisse zeigen, daß die DNA-Extraktion aus diesem Probenmaterial relativ schonend erfolgen kann. Drastische Bedingungen wie erhöhte Temperatur und Alkali erhöhten in erster Linie den Anteil an hochmolekularen, möglicherweise die Polymerase-Kettenreaktion störenden, Farbstoffe.

### 5. DNA-Extraktion aus Maisstärke

1 g Maisstärke wurde in 1 ml Wasser (reinst) suspendiert und 1 min auf 90°C erhitzt, wobei sich ein fester halb-transparenter Block bildete. Dieser kann zur Verbesserung der Extraktion gegebenenfalls mechanisch zerkleinert werden. Nach Zusatz von 2 mg α-Amylase (Roche) und 10 mg Cellulase bei 50°C wurde der Aufschluß 3 Stunden bei dieser Temperatur gedreht, wodurch sich der größte Teil des Blockes verflüssigte. Die enzymatische Behandlung kann gegebenenfalls auf 8 bis 10 Stunden ausgedehnt werden. Nach Zusatz von 1.5 ml Gua-Puffer (ohne Triton X100) und 80 µl 8 M NaOH wurde 5 min auf 95-100° erhitzt (es trat eine Gelbfärbung auf) und dann 10 min bei 4000 UpM (Hettich-Zentrifuge, Rotor 1624) zentrifugiert. Der Überstand wurde in 0.3 M NaCl/0.3 M NaOH bei ausreichendem Überdruck für eine Flußrate von 0.3-0.5 cm/min durch die oben in III. 2. beschriebene BiOSEC/TMAE-Säulenkombination filtriert, worauf mit 1 ml Laufmittel (0.3 M NaCl/0.3 M NaOH) nachgespült wurde. Die Desorption/Elution von der TMAE-Säule erfolgte wie oben beschrieben mit je 80 µl 0.7 M NaCl, 0.9 M NaCl und 1.2 M NaCl, jeweils in 0.3 M NaOH.

Die nach dem Umpuffern in TE-Puffer (Microspin) aufgenommenen UV-Spektren waren zwar nicht aussagekräftig, jedoch ergaben sich PCR-Amplifikate, was bedeutet, daß der Hauptteil der im UV-Bereich absorbierenden Komponenten DNA war.

## Patentansprüche

1. Verfahren zur Isolierung, und gegebenenfalls Größenfraktionierung, von einzelsträngiger DNA aus einem Probenmaterial, bei dem man
a) das DNA-haltige Probenmaterial mit einem alkalischen Medium bei pH > 12 behandelt,
b) in diesem Medium mit einem Anionenaustauscher in Kontakt bringt, der auf inerten Trägerteilchen kovalent daran gebundene, unvernetzte hydrophile Linear-Polymere mit eingebauten Tetraalkylammoniumgruppen aufweist, und
c) die an den Anionenaustauscher gebundene DNA im alkalischen Medium von diesem eluiert.

2. Verfahren nach Anspruch 1, wobei das alkalische Medium ein Alkalihydroxid in einer Konzentration im Bereich von 0.1 M bis 1 M enthält.

3. Verfahren nach Anspruch 2, wobei das Alkalihydroxid NaOH oder KOH ist.

4. Verfahren nach einem der vorstehenden Ansprüche, wobei zur Elution Salz einer Konzentration im Bereich von 0.6 bis 1.2 M oder ein Salzgradient im Bereich von 0.6 bis 1.2 M verwendet wird.

5. Verfahren nach Anspruch 4, wobei Salz einer Konzentration im Bereich von 0.7 bis 0.9 M verwendet wird.

6. Verfahren nach einem der Ansprüche 4 oder 5, wobei das Salz NaCl oder KCl ist.

7. Verfahren nach einem der vorstehenden Ansprüche, wobei es sich bei dem Probenmaterial um Nähr- oder Nahrungsmittel pflanzlichen, tierischen oder mikrobiellen Ursprungs handelt.

8. Verfahren nach Anspruch 7, wobei das Nähr- oder Nahrungsmittel ausgewählt ist unter Kartoffeln, Tomatenmark, Orangensaft-Konzentrat, Lecithin, Schokolade, Nougat-Creme, Mais- oder Sojamehl, Fleisch und Wurstprodukten.
